# EUROPEAN PATENT APPLICATION

(11) **EP 2 085 462 A1**
(43) Date of publication of application: **05.08.2009**
(21) Application number: 08001919.3
(22) Date of filing: 01.02.2008
(51) Int. Cl.: C12M 1/18, C12M 1/22

(54) **Cell culture dish**

(71) Applicant: EPPENDORF AG, 22339 Hamburg (DE)
(72) Inventor: Beese, Jochen, 22848 Norderstedt (DE); Haker, Ute, Dr., 22083 Hamburg (DE)
(74) Representative: Becker Kurig Straus

(57) **Abstract**

The present invention relates to a to a cell culture dish and particularly to a Petri dish. In the present cell culture dish the wall portion of the base and the top portion of the cover are both provided with markings enabling the correlation of particular cell growth areas to particular coordinates permitting retrieval of particular growth areas avoiding disturbing markings provided on the bottom of the dish.

## Description

The present invention relates to a to a cell culture dish and particularly to a Petri dish. In the present cell culture dish the wall portion of the base and the top portion of the cover are both provided with markings enabling the correlation of particular cell growth areas to particular coordinates permitting retrieval of particular growth areas avoiding disturbing markings provided on the bottom of the dish.

Cell culture refers to a process in which prokaryotic or eukaryotic cells are grown/proliferated under controlled conditions. Particularly, growth of eukaryotic cells, such as cells derived from animals, plants, fungi, and protists, poses a challenge due to different conditions and techniques required for e.g. Isolation of cells, maintaining in culture, manipulation, Media changes, passaging cells, transfection and transduction, establishing of particular cell lines, such as hybridoma. Culturing of the cells is often performed on solid medium in particular dishes.

These dishes have, however, the disadvantage that an unambiguous correlation of a particular cell or cell area to a particular location of the dish permitting retrieval of the cell/cell area is not possible.

In order to overcome this disadvantage, several solutions have been proposed in the art.

US 5,380,493 pertains to a device for holding a laboratory cell well plate in a more accessible and visible position and for indicating which individual cell wells have been used or accessed during the experimental session. The device comprises an angled receiving face that supports and retains a cell well plate and an electronic array of detectors and indicators for identifying the use of specific cell wells on the cell well plate. Said device is, however, not applicable to all kind of culture dishes.

A similar device for Petri dishes is disclosed in US 5,747,333. Said document discloses a support device comprising two opposed portions connected by e.g. a hinge joint, and a space defined between said opposed portions in which a sheet of material having marked thereon a matrix may be inserted. The support may be adapted to secure a Petri dish above the marked matrix in that the matrix is visible through the base of the Petri dish.

Other solutions reside in providing markers on certain areas of cell culture dishes and are e.g. outlined in US 5,928,858. On the lower surface of the bottom portion of a Petri dish a removable marking element defining a plurality of predetermined locations is applied with the marking element being visible through the upper surface. The removable marking element enables correlation between microorganisms within the base unit and one of the predetermined locations. The removable marking element may include a several dividing lines defining locations, which may be a plurality of squares in a grid configuration or a plurality of pie-shaped sectors.

Other cell culture dishes providing correlation of a particular cell or cell area to a particular location of the dish are conventional micro titer plates, special dishes for counting cells in which the bottom of the base is provided with a grid optionally provided with alphanumerical signs and Petri dishes exhibiting zoning by walls formed on the bottom of the base. Such culture dishes are inter alia described in conventional catalogues for laboratory apparatuses, such as by Greiner (Kremsmünster, Austria).

The solutions proposed so far have, however, the disadvantage that either a particular device is necessary in order to allocate cells to a particular location, or that the markings are provided in a location disturbing during collection of particular cells.

The problem of the present inventions resides in overcoming the disadvantages of the cell culture dishes according to the prior art and to provide cell culture dishes with markings, which does not interfere with manual and/or automatic detection and/or collection of a cell, colonies or cell areas in general.

The above problem has been solved by providing the present culture dish for culturing cells in which both, the top portion of the cover and the wall portion of the base, are provided with markings in that in case of e.g. a Petri dish, the user may turn the cover and allocate the markings thereon with the markings on the base's wall portion, forming e.g. a circular coordinate system in which a particular position is absolutely defined on the one hand on the distance from the center of the Petri dish and on the other hand on circular coordinates provided in form of e.g. equidistantly spaced markings on the wall portion of the base. Another advantage resides in that with the present markings higher organization degrees may be obtained as the markings on the cover may be in form of a line or ruler and the wall of the base both with even high numbers of alphanumeric characters. As the cover is on the one hand removed during collection of a cell and the wall of the base is not perceivable in the top view, said markings will not disturb during collection/detection of a cell.

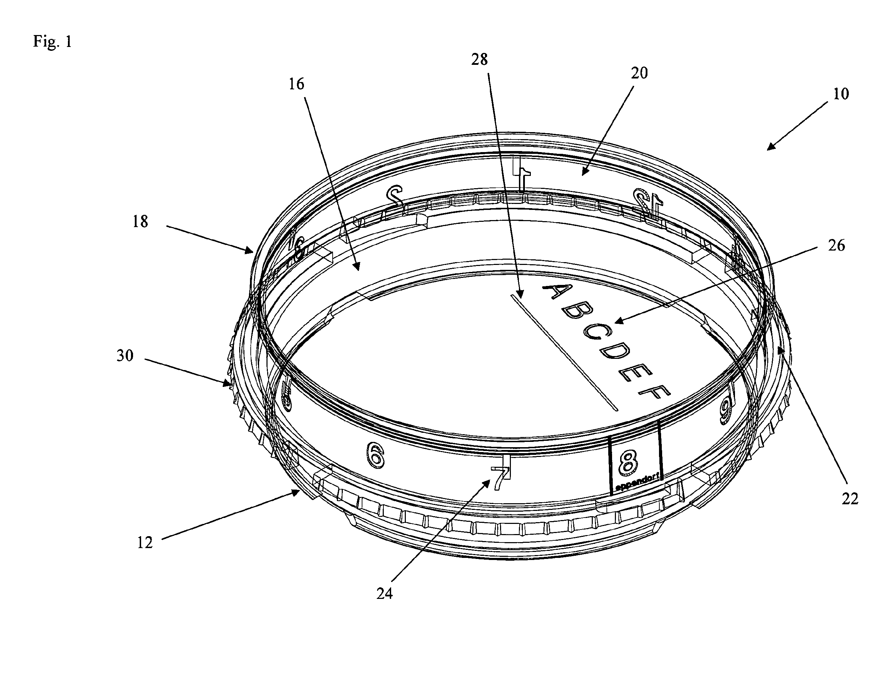

In the Figures, shows Fig. 1 a preferred embodiment of a cell culture dish (10) according to the present invention. The base (12) is formed of a bottom portion and a first wall portion (16) extending upward therefrom. The lid or cover (18) has a top portion (20) and a second wall portion (22) extending downward therefrom and is adapted to cover the base. The first wall portion is provided with numbers (24) ranging from 1 to 12 forming the first markings. The second markings are in form of the letters (26) A, B, C, D, E and F and a ruler (28) arranged in vicinity, which constitutes the radius of the top portion. The lid is provided with a gripping area (30) facilitating handling and accurate rotation of the lid in order to allocate a characterizing letter and number combination to e.g. a particular cell grown in the culture dish.

According to a first embodiment of the present invention a cell culture dish is provided. The dish is made of a substantially transparent material and comprises a base having a bottom portion and a first wall portion extending upward therefrom, a cover having a top portion and a second wall portion extending downward therefrom, wherein said cover is adapted to cover said base. The first wall portion is further provided with first markings thereon and the top portion is provided with second markings thereon.

The present cell culture dish may have any basic shape. Suitable shapes comprise e.g. circular, rectangular, quadratic or other symmetrical shapes. An example of a circular cell culture dish is a Petri dish, whereas an example of a cell culture dish with a rectangular basic shape is a micro titer plate or multi-well plate. The dish may be manufactured from the materials apparent to persons of skill in the art, such as for example polystyrene, polyethylene, polypropylene, polycarbonate, and polyvinyl thermoplastic resins using conventional injection-molding or thermoforming methods. Another suitable material is glass. Depending on the intended uses, the test culture dish may be manufactured to contain one chamber or a plurality of chambers. For example, a multi-chamber (multi-well) cell culture dish of the invention may contain for example 4, 6, 8, 12, 24, 48, 96, 384 or 1536 chambers (wells) per dish. Depending upon the size of chambers in the cell culture dish, the capacity of each chamber may vary; for example the chamber may contain from e.g. about 10 ml to about 5 nl. The dimension of cell culture dishes and their fabrication are well known in the art. The lid of the culture dish may be also provided with a gripping area.

It will be appreciated that the use of the present cell culture dish is not limited to any particular use. A cell culture dish according to the present invention may be employed for example for routine experiments, in vivo tests, assaying adherence of cells and effect of chemical substances, such as growth factors, on cells.

The dishes are made out of a substantially transparent material, such as glass or the plastics indicated above. Substantial transparent is direct to a transparency of the material still permitting visual detection of cell areas and colonies from outside the closed dish. In particular, said term is directed to a transparency of 50% or more.

It will be appreciated that the cover may have the same basic shape as the base. Preferably the cover has dimensions in comparison to the base permitting complete and sealing cover of the latter. The term sealing cover is directed to both to hermetic sealing but also to a sealing still permitting gas and humidity exchange between the interior of the dish and the environment as in conventional Petri dishes intended for the growth of cells under aerobic conditions. In such a case, sealing is preferably obtained by forming small elevations, such as tappets, on the outside of the first wall portion and/or the inside of the second wall portion. Sealing may be however substantially complete in case anaerobic conditions for cell growth are envisaged.

The first and second markings may be in form of rulers, which are equidistantly provided with alphanumerical characters in order to create a coordinate system, in which one coordinate is preferably provided with numbers and the other with letters. The markings may be provided with e.g. conventional casting techniques, or by printing, gluing, etc. on the surface of the cell culture dish. It will be understood that the markings may have any shape, color and may also form elevations or recesses in the dish. Additionally, markings may have a transparency different from that of the dish material. Preferably, the markings exhibit a lower transparency than the dish material, such as 0, 10, 20, 30 or 40%.

According to another embodiment, a cell culture dish is provided, wherein the substantially transparent material is made of plastics or glass. Examples of suitable plastics comprise polystyrene, polyethylene, polypropylene, polycarbonate, and polyvinyl thermoplastic resins, which are formed by applying conventional injection-molding or thermoforming methods. It will be appreciated that the material of the culture dish is dependent from the later application. It may be intended for example, to conduct an assay in said dish requiring a particular chemical compound reacting with specific plastics. In such a case e.g. glass may be used.

According to still another embodiment said bottom portion and said top portion have both a circular area and form preferably a Petri dish. In a Petri dish the markings may be provided in a manner extending around the entire wall of the base, and in form of a radiant on the top of the cover. By turning the cover and base in opposite directions, all individual locations on the dish may be determined / detected as the markings provide the minimal requirements for a circular coordinate system, which may be defined by a position on the radiant of the circle and an angle.

According to an embodiment of the present invention said first wall portion is tapered upwards and said second wall portion is tapered downwards in order to facilitate removal of the cover / lid.

In another embodiment the first markings provided on the first wall portion are equidistantly spaced from each other forming in case of a Petri dish a complete circle of 360°, wherein e.g. in each 5, 10, 20, 30, 40, 60, 90, 120 or 180 degrees a marker is provided, in that on the top of the cover merely a ruler in form of a radiant is required. By turning of the lid, different first and second markings may be correlated to allocate to each point on the base' surface two coordinates.

In case of a dish having a square base shape, the first side walls have markings provided thereon. As also the cover of said dish may be turned for a 90° angle (by lifting the cover and turning) a quarter of the top has to be provided with any markings in order to provide on each point on the base' surface two coordinates.

Similar symmetrical approaches apply to other conceivable base shapes having a symmetry axis (a Cₙ axis with n = any natural number greater than 1) extending orthogonally from center of the dish plane.

According to still another embodiment the first markings are equidistantly spaced from each other providing a measure for allocating the position of e.g. a cell or colony to a first coordinate.

According to an embodiment the second markings are in form of a ruler. The ruler may be e.g. in form of a line having equidistant markings thereon. The ruler may be also in form of a rectangular or pie-like area. In any case the ruler will cover a small part of the top portion bestowing visibility of the dish's interior. It will be appreciated that by turning of the cover other parts of the dish's interior may be visible.

According to a preferred embodiment the second markings are provided in an area exhibiting a magnification function. Such a magnification function may be provided by means of a lens formed in the top portion. A preferred kind of lens is a Fresnel lens comprising a number of concentric circles, which is thinner and flatter than a conventional lens. Lenses, their preparation, as well as applying or fastening to an area of the top portion are known in the art.

According to still another embodiment said first and second markings are provided with signs. Said signs are preferably in form of alphanumerical signs, in that e.g. the first markings are provided with numbers and the second markings with alphabetic characters or vice versa. This provides the possibility to allocate to any cell or (cell) colony in the dish two coordinates facilitating retrieval and reproducibility of cells or colonies.

In another embodiment, a method for preparing the present cell culture dish is provided. Said method comprises the steps of providing a cell culture dish comprising a base having a bottom portion and a first wall portion extending upward therefrom and a cover having a top portion and a second wall portion extending downward therefrom, wherein said cover is adapted to cover said base, and casting of markings on the first and second wall portions. It will be appreciated that the markings may be alternatively provided during the manufacturing process. Of advantage is hereby that conventional devices for manufacturing may be employed. It will be appreciated that the use of such devices as well as the required materials are within the knowledge of the skilled person.

Although preferred embodiments of the disclosed invention have been described and shown herein, it will be apparent to those skilled in the art, that aspects of the disclosed invention may be modified.

## Claims

1. A cell culture dish made of a substantially transparent material, comprising:
a base having a bottom portion and a first wall portion extending upward therefrom,
a cover having a top portion and a second wall portion extending downward therefrom, wherein
said cover is adapted to cover said base, **characterized in that**
the first wall portion is provided with first markings thereon and that the top portion is provided with second markings thereon.

2. Cell culture dish according to claim 1, wherein the substantially transparent material is made of plastics or glass.

3. Cell culture dish according to any of the preceding claims, wherein said bottom portion and said top portion have a circular area.

4. Cell culture dish according to claims 2 and 3, which is in form of a Petri dish.

5. Cell culture dish according to any of the preceding claims, wherein said first wall portion is tapered upwards and said second wall portion is tapered downwards.

6. Cell culture dish according to any of the preceding claims, wherein the first markings are equidistantly spaced from each other.

7. Cell culture dish according to any of the preceding claims, wherein the second markings are in form of a ruler.

8. Cell culture dish according to any of the preceding claims, wherein the second markings are provided in an area exhibiting a magnification function.

9. Cell culture dish according to any of the preceding claims, wherein said first and second markings are provided with signs.

10. Cell culture dish according to claim 9, wherein said signs are alphanumerical signs.

11. Cell culture dish according to claim 9 or 10, wherein the first markings are provided with numeric signs and said second markings are provided with alphabetic characters.

12. A method for preparing a cell culture dish, comprising the steps of:
providing a cell culture dish comprising a base having a bottom portion and a first wall portion extending upward therefrom and a cover having a top portion and a second wall portion extending downward therefrom, wherein said cover is adapted to cover said base, and
casting of markings on the first and second wall portions.
